# EUROPÄISCHE PATENTSCHRIFT

(11) **EP 1 683 781 B1**
(45) Veröffentlichungstag und Bekanntmachung des Hinweises auf die Patenterteilung: **01.01.2014**
(21) Anmeldenummer: 06000243.3
(22) Anmeldetag: 07.01.2006
(51) Int. Cl.: C08G 63/676, C08G 63/668, C07C 69/732, A61K 8/85, A61Q 19/00, A61K 47/14, B01F 17/00

(54) **Polyglycerinpartialester von Polyricinolsäure und mehrfunktionellen Carbonsäuren und deren Verwendung zur Herstellung von Emulsionen und Dispersionen**
Polyglycerine partial esters of polyricinoleic acid and polycarbonxylic acids and their use in the preparation of emulsions and dispersions
Esters partiels du polyglycérol avec l'acide polyricinoléique et des acides polycarboxyliques et leur utilisation pour la préparation d'émulsions et de dispersions

(30) Priorität: 21.01.2005 DE 102005003164
(43) Veröffentlichungstag der Anmeldung: 26.07.2006
(73) Patentinhaber: Evonik Goldschmidt GmbH, 45127 Essen (DE)
(72) Erfinder: Allef, Petra Dr., 45147 Essen (DE); Berkels, Wolfgang, 46239 Bottrop (DE); Fötsch, Hannelore, 45355 Essen (DE); Meyer, Jürgen Dr, 48143 Münster (DE)

(56) Entgegenhaltungen:
- EP-A1- 0 835 862
- DE-A1- 4 420 516
- DE-C1- 4 409 569

## Beschreibung

Die Erfindung betrifft Polyglycerinpartialester von Polyricinolsäure und mehrfunktionellen Carbonsäuren, erhältlich durch Veresterung eines Polyglyceringemisches mit Polyricinolsäure und mit aliphatischen, linearen oder verzweigten Dicarbonsäuren mit einer Kettenlänge von 2 bis 36 C-Atomen und gesättigten oder ungesättigten, linearen oder verzweigten Fettsäuren mit 6 bis 22 C-Atomen, wobei der Veresterungsgrad der Polyglycerinmischung zwischen 20 und 75 % liegt und deren Verwendung zur Herstellung von Emulsionen und Dispersionen für insbesondere kosmetische und pharmazeutische Formulierungen.

Es ist dem Fachmann bekannt, dass Wasser-in-Öl-Emulsionen aufgrund der dichten Packung der Wassertröpfchen, die eine Folge des üblicherweise hohen Anteils an disperser Phase (> 65 %) ist, schwer gegen Koaleszenz der Wassertröpchen und damit gegen Wasserabscheidung zu stabilisieren sind. Eine weitere Folge der hohen Packungsdichte ist eine per-se hohe Viskosität der Emulsion.

Um eine von der kosmetischen Industrie geforderte Stabilität zu erzielen, verwendet man in der Regel stabilisierende Wachse und zum Teil auch hochviskose Öle, die beide die Viskosität der (äußeren) Ölphase erhöhen, somit die Mobiliät der Wassertröpfchen reduzieren und gegen Koaleszenz schützen. Hochviskose Öle und stabilisierende Wachse haben jedoch einen negativen Einfluss auf das Hautgefühl, was sich in einem schweren und klebrigen Hautgefühl bemerkbar macht. Zwar kann man grundsätzlich auf Öl-in-Wasser-Emulsionen, die bekanntlich ein leichteres Hautgefühl besitzen, als Alternative ausweichen, allerdings besitzen diese einen deutlich geringeren Pflegeeffekt als Öl-in-Wasser-Emulsionen.

Ein allgemein erstrebenswertes Ziel ist es also, Wasser-in-Öl-Emulsionen möglichst so leicht, d.h. niedrigviskos, wie Öl-in-Wasser-Emulsionen zu machen, ohne deren besonderen Pflegeeffekt aufgeben zu müssen. Aufgabe ist es insbesondere, möglichst niedrigviskose, nicht klebrige und trotzdem stabile Wasser-in-Öl-Emulsionen zu ermöglichen. Eine besondere Herausforderung in diesem Bereich stellt die Formulierung von Öl-in-Wasser-Emulsionen dar, die so niedrigviskos sind, dass sie sprühbar sind. Hierbei kommt dem zu verwendenden Emulgator eine besondere Schlüsselrolle zu.

An W/O-Emulgatoren, die auf nativen Rohstoffen basieren, besteht aus ökologischen Gründen sowohl bei den Produzenten als auch bei den Konsumenten von Emulsionspräparaten ein erhebliches Interesse. Dabei handelt es sich insbesondere um Emulgatoren, die keine polyethylenglykolhaltigen Reste enthalten ("PEG-freie" Emulgatoren). Aus diesem Grunde finden, trotz ihrer oft mittelmäßigen Leistungsfähigkeit, Partialester aus Polyalkoholen, wie Glycerin, Polyglycerin, Sorbitol oder Methylglycosid und Fettsäuren, wie Öl- oder Isostearinsäure, nach wie vor eine vielfältige Verwendung.

Dieser Emulgatortyp ist z. B. für fließfähige Emulsionen (Lotionen) und für Cremes mit hohem Gehalt an nativen Triglyceriden nicht geeignet. Die den Stabilitätsanforderungen des Marktes entsprechenden Cremes (Temperaturbelastbarkeit von -15 bis +45 °C, teilweise von -25 bis +50 °C) enthalten an lipidartigen Komponenten überwiegend Paraffinöle und Fettsäureester von Monoalkanolen (MG < 500); diese haben günstigere technologische Eigenschaften als die höhermolekularen Triglyceride. Zur Stabilisierung werden dennoch relativ hohe Konzentrationen an viskositätserhöhenden Wachsen (≥ 3 %) benötigt, die sich jedoch negativ auf die Applikationseigenschaften auswirken, da sie ein unerwünschtes klebrig-fettiges Gefühl auf der Haut erzeugen.

Erheblich bessere Emulgiereigenschaften als die Polyalkohol-Fettsäurepartialester besitzen die Polyglycerinester di- und polymerisierter ungesättigter C₁₈-Fettsäuren. Sie werden aus den Mono- und Diglyceriden von Pflanzenölen, bevorzugt Sojaöl, durch eine mehrstündige thermische Behandlung bei ca. 300 °C bzw. durch die Umesterung eines thermisch polymerisierten Pflanzenöls mit Polyglycerin gewonnen.

Die nach einem analogen Verfahren aus Rizinusöl gebildeten Polyglycerin-Polyricinoleate sind ebenfalls leistungsfähige W/O-Emulgatoren (DE-B-44 09 569).

Insbesondere aufgrund ihrer Oxidationsempfindlichkeit und des teilweise ausgeprägten fettig-ranzigen Geruchs konnte sich diese Substanzklasse bisher nur in wenigen kosmetischen oder pharmazeutischen Emulsionspräparaten etablieren. Verantwortlich dafür sind in erster Linie die massive thermische Belastung während der Herstellung und der ungesättigte Charakter (Jodzahl ca. 100).

Das dem Polyglycerin-Polyricinoleat chemisch verwandte, ebenfalls aus vegetabilen Rohstoffen herstellbare Polyglycerin-Polyhydroxystearat wird ebenfalls als W/O-Emulgator verwendet, besitzt eine höhere Oxidationsstabilität und damit eine erhöhte sensorische Qualität (DE-C-44 20 516).

Beide Substanzklassen sind jedoch nur in der Lage, relativ dünnflüssige W/O-Lotionen mit einem Ölphasengehalt > 22 Gew.-% zu bilden. Aufgrund dieser Mindestmenge an Ölphase und aufgrund des eher reichhaltigen sensorischen Charakters dieser Emulsionen, ist die Formulierung stabiler W/O-Emulsionen mit einem sehr leichten Hautgefühl nicht oder nur sehr eingeschränkt möglich. Hinzu kommt, dass Emulgatoren dieses Typs insbesondere bei der Formulierung von triglyceridhaltigen Formulierungen, oft Probleme in der Kältestabilität aufweisen, die in vielen Fällen nur durch Zusatz von Co-Emulgatoren gelöst werden können.

Die Verbesserung der Gefrier-Tau-Belastbarkeit ist für die Transport- und Lagerfähigkeit der Emulsionspräparate von erheblichem praktischen Interesse. Durch längere Lagerung bei sehr tiefen Temperaturen oder durch extreme Temperaturschwankungen bei längeren Transportwegen kann sich die ungenügende Emulsionsstabilisierung durch deutliche Wasserabscheidungen im Emulsionspräparat zeigen oder sogar zum völligen Brechen der Emulsion führen.

In EP-B-0 835 862 werden Polyglycerinpartialester beschrieben, die erhältlich sind durch Veresterung eines Polyglyceringemisches mit einem Veresterungsgrad des Polyglycerins zwischen 30 und 75 % und gesättigten oder ungesättigen, linearen oder verzweigten Fettsäuren mit 12 bis 22 C-Atomen und Dimerfettsäuren mit einer mittleren Funktionalität von 2 bis 2,4. Diese besitzen gegenüber dem Polyglycerin-Polyhydroxystearat den zusätzlichen Vorteil, dass insbesondere die Gefrierstabiltität dieser Emulsionen deutlich verbessert ist. Die Emulsionen sind allerdings noch relativ dickflüssig, weswegen diese Polyglycerinpartialester sich hauptsächlich zur Herstellung reichhaltiger Lotionen und Cremes eignen.

Die Aufgabe der Erfindung bestand daher darin, einen PEG-freien Emulgator zu entwickeln, der die Formulierung von dünnflüssigen Emulsionen erlaubt, die ein angenehm leichtes Hautgefühl und gleichzeitig die geforderte Gefrier-Tau-Belastbarkeit aufweisen.

Überraschenderweise wurde nun gefunden, dass sehr dünnflüssige, leichte Wasser-in-Öl-Emulsionen durch Emulgatoren auf der Basis von Polyglycerinpartialestern von Polyricinolsäure, die zusätzlich über Di- oder Tricarbonsäuren verknüpft sind, erhalten werden können. Diese verknüpften Polylglycerin-Polyricinoleate besitzen weder einen fettig-ranzigen Geruch, noch zeigen sie eine stärkere Oxidationsempfindlichkeit als Polyglycerin-Polyhydroxystearate. Mit Hilfe dieser Emulgatoren ist es möglich, im Vergleich zu nicht vernetzten Polyglyceryl-Ricinoleaten und - Polyhydroxystearaten Emulsionen mit deutlich niedrigerer Viskosität zu formulieren. Dies ermöglicht es, Lotionen mit einem sehr niedrigen Ölphasengehalt (< 22 Gew.-%) zu formulieren, die ein dementsprechend leichtes Hautgefühl besitzen.

Ein Gegenstand der Erfindung sind daher Polyester von Polyricinolsäure und mehrfunktionellen Carbonsäuren, erhältlich durch Veresterung
a) eines Polyglyceringemisches mit
b) mindestens einer Polyricinolsäure der allgemeinen Formel 1 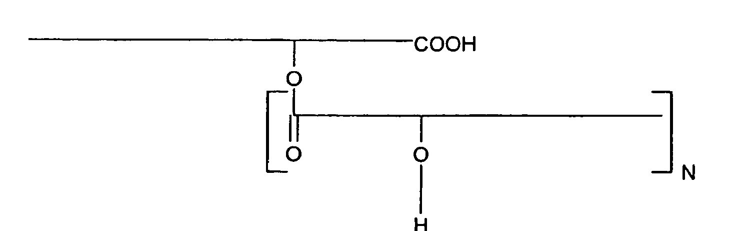 worin 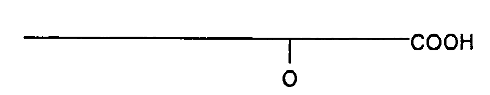 der Rest der Ricinolsäure CH₃-(CH₂)₅-CH(OH)-CH₂-CH=CH-(CH₂)₇-COOH und gegebenenfalls der Hydroxystearinsäure CH₃- (CH₂)₅-CH(OH) - (CH₂)₁₀-COOH ist, wobei die Homopolymerisate auf Basis der Ricinolsäure bevorzugt sind,
   N 1 bis 10, vorzugsweise 2 bis 8, insbesondere 2 bis 5 bedeutet und gegebenenfalls
b1) Polyhydroxystearinsäure und
c) mindestens einer Di- und/oder Tricarbonsäure und
d) mindestens einer Fettsäure mit 6 bis 22 C-Atomen nach an sich bekannten Verfahren.

Ein weiterer Gegenstand der Erfindung sind Polyester von Polyricinolsäure und mehrfunktionellen Carbonsäuren, erhältlich durch Veresterung

Ein weiterer Gegenstand der Erfindung sind Polyglycerinpartialester gemäß mindestens einem der Ansprüche 1 bis 6, hergestellt durch Veresterung von
a) 1,0 Mol OH-Gruppen des Polyglyceringemisches mit
b) 0,01 bis 0,5 Mol, insbesondere 0,05 bis 0,3 Mol COOH-Gruppen der Polyricinolsäure und
c) 0,01 bis 0,5 Mol, insbesondere 0,05 bis 0,3 Mol COOH-Gruppen mindestens einer Di- und/oder Tricarbonsäure und
d) 0,01 bis 0,9 Mol, insbesondere 0,1 bis 0,6 Mol COOH-Gruppen der Fettsäuren mit 6 bis 22 C-Atomen,
mit der Maßgabe, das die Summe der COOH-Gruppen etwa 20 bis 75 % der OH-Gruppen des Polyglyceringemisches entspricht.

Ein weiterer Gegenstand der Erfindung sind kosmetische, dermatologische oder pharmazeutische Zubereitungen, die mindestens einen der erfindungsgemäßen Polyglycerinpartialester enthalten. Bei diesen Zubereitungen handelt es sich dabei vorzugsweise um Emulsionen, die gegebenenfalls auch dispergierte Feststoffe enthalten können. Insbesondere steht dabei der Einsatz der erfindungsgemäßen Polyglycerinpartialester als Wasser-in-Öl-Emulgatoren oder Dispergierhilfsmittel im Vordergrund.

Ein weiterer Gegenstand der Erfindung sind Pflege- und Reinigungsmittel für Haushalt und Industrie, insbesondere für harte Oberflächen, für Leder oder Textilien, die mindestens einen der erfindungsgemäßen Polyglycerinpartialester enthalten. Bei diesen Zubereitungen handelt es sich vorzugsweise um Emulsionen, die gegebenenfalls auch dispergierte Feststoffe enthalten können. Insbesondere steht dabei der Einsatz der erfindungsgemäßen Polyglycerinpartialester als Wasser-in-Öl-Emulgatoren oder Dispergierhilfsmittel im Vordergrund.

Die erfindungsgemäßen Kondensationsprodukte auf Basis der Ricinolsäure sind flüssig und eignen sich somit neben der klassischen "Heiß-Heiß"-Herstellung auch zur energiesparenden "Kalt-Kalt"-Herstellung von Emulsionen.

Die Herstellung der erfindungsgemäß mitverwendeten Polyricinolsäuren erfolgt nach den bekannten Verfahren des Standes der Technik, beispielsweise durch Polykondensation von Ricinolsäure, gegebenenfalls unter Mitverwendung von Hydroxystearinsäure.

Es werden Veresterungsprodukte der allgemeinen Formel 1 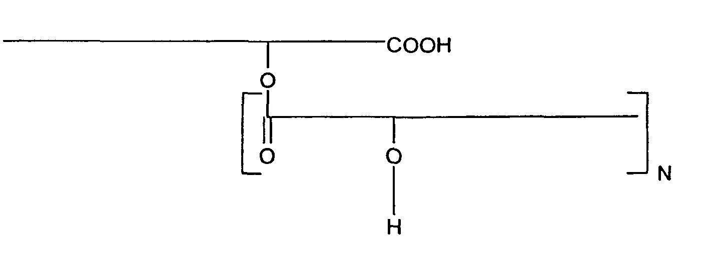 mit
N 1 bis 10, vorzugsweise 2 bis 8, insbesondere 2 bis 5 Fettsäureeinheiten gebildet, die Säurezahlen zwischen 187 und 20, bevorzugt zwischen 96 und 45 aufweisen und worin
der Rest 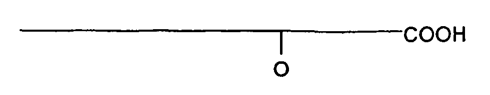 vorzugsweise nur der Rest der Ricinolsäure CH₃-(CH₂)₅₋CH (OH) -CH₂-CH=CH- (CH₂)₇-COOH ist,
hergestellt.

Erfindungsgemäß können auch Mischungen aus Polyricinolsäure und Polyhydroxystearinsäure eingesetzt werden.

Als Polyglycerinc sind insbesondere solche der allgemeinen Formeln 2A und 2B 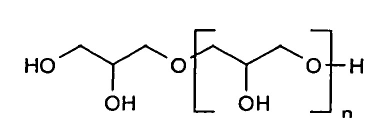 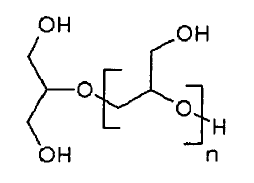 mit einem mittleren Kondensationsgrad n von 1 bis 11, bevorzugt 2 bis 6 und Hydroxylzahlen von ca. 1.350 bis ca. 800, bevorzugt ca. 1.200 bis ca. 900 geeignet. Die Polyglyceringemische können auch andere Regioisomere oder Zyklen enthalten.

Dabei handelt es sich um technische Polyglycerinmischungen, die z. B. durch alkalisch katalysierte Kondensation von Glycerin bei erhöhten Temperaturen erhalten werden, aus denen sich gegebenenfalls durch Destillationsverfahren Fraktionen mit dem erwünschten Kondensationsgrad erhalten lassen. Ebenfalls geeignet sind auch PoIyglycerine, die auf anderem Wege, z. B. aus Epichlorhydrin oder Glycidol gewonnen werden.

Als besonders vorteilhaft hat sich die Verwendung von Polyglycerinen erwiesen, die die folgende Homologenverteilung (GC-Methode) aufweisen; in Klammern angegeben sind die bevorzugten Bereiche:
- Glycerin:: 0 bis 20 (< 5) Gew.-%
- Diglycerine:: 0 bis 60 (5 bis 30) Gew.-%
- Triglycerine:: 0 bis 60 (5 bis 50) Gew.-%
- Tetraglycerine:: 0 bis 30 (5 bis 25) Gew.-%
- Pentaglycerine:: 0 bis 30 (5 bis 20) Gew.-%
- Oligoglycerine:: ad 100 Gew.-%

Besonders geeignet für den erfindungsgemäßen Einsatzzweck als Emulgatoren ist die Verwendung von relativ kurzkettigen Di- oder Tricarbonsäuren, wie Oxalsäure, Malonsäure, Bernsteinsäure, Glutarsäure, Adipinsäure, Pimelinsäure, Korksäure, Azelainsäure, Sebacinsäure, Dodecandisäure. Ebenso geeignet sind Hydroxydi- bzw. tricarbonsäuren, wie Äpfelsäure, Weinsäure, Zitronensäure. Desweiteren können aromtische Säuren, wie Phthalsäure, Isophthalsäure oder Terephhtalsäure verwendet werden.

Die zur Herstellung der erfindungsgemäßen Verbindungen gegebenenfalls mitverwendeten dimeren Fettsäuren sind die handelsüblichen Produkte, welche durch Polymerisation von gesättigten oder ungesättigten natürlichen oder synthetischen einbasischen aliphatischen Fettsäuren mit 16 bis 22 Kohlenstoffatomen nach bekannten Methoden hergestellt werden (vgl.-US-2 482 761, US-3 256 304). Typische im Handel erhältliche dimere Fettsäuren haben etwa folgende Zusammensetzung:
- Monomere Säuren: 0 bis 15 Gew.-%
- dimere Säuren: 60 bis 95 Gew.-%
- tri- und höher:
- polymerisierte Säuren: 1 bis 35 Gew.-%
wobei der Gehalt je nach Herkunft der Monomeren, des Polymerisationsverfahrens sowie des Aufbereitungsprozesses innerhalb dieser Grenzen schwanken kann.

Die eingesetzte dimere Fettsäure kann auch in hydrierter Form vorliegen.

Der Gehalt an dimerer Säure kann durch allgemein bekannte Destillationsverfahren bis zu 100 Gew.-% erhöht werden. Er wird bestimmt nach den bekannten Verfahren der Gas-Liquid-Chromatography (GLC). Die Säurezahlen liegen im Bereich von ca. 190 bis 200.

Durch Mitverwendung dieser Säuren kann nochmals eine deutlich verbesserte Stabilisierung der Phasengrenzflächen in W/O-Emulsionen erzielt werden.

Als zusätzliche Fettsäurekomponenten eignen sich vor allem gesättigte Fettsäuren, wie Laurinsäure, Tridecansäure, Myristinsäure, Palmitinsäure, Margarinsäure, Stearinsäure, Isostearinsäure, Arachinsäure und Behensäure sowie Mischungen hieraus.

Natürlich vorkommende Mischungen sind beispielsweise die Kokosfettsäuren, die als Hauptbestandteil Laurinsäure, daneben noch gesättigte C₁₄-C₁₈-Fettsäuren und gegebenenfalls gesättigte C₈-C₁₀-Fettsäuren und ungesättigte Fettsäuren enthalten, sowie Talgfettsäuren, welche im Wesentlichen eine Mischung aus Palmitinsäure und Stearinsäure darstellen.

Als zusätzliche ungesättigte Fettsäurekomponenten eignen sich monoolefinisch ungesättigte Säuren, beispielsweise Hexadecensäuren, Octadecensäuren, wie Ölsäure (cis-9-Octa-decensäure) oder Elaidinsäure (trans-9-Octadecensäure), Eicosensäuren und Docosensäuren, wie Erucasäure (cis-13-Docosensäure) oder Brassidinsäure (trans-13-Docosensäure), mehrfach ungesättigte Fettsäuren, beispielsweise Octadecadiensäuren und Octadecatriensäuren, wie Linolsäure und Linolensäure, sowie Mischungen hieraus.

Besonders geeignet sind die flüssigen Fettsäuren wie Öl-, Ricinol-, Eruca- und Isostearinsäure, die 18 bis 22 Kohlenstoffatome enthalten. Ihre Erstarrungspunkte liegen aufgrund einer Verzweigung oder einer Doppelbindung in der Kohlenwasserstoffkette unter 35 °C. Verwendbar sind weiterhin Fettsäuregemische, die auch wachsartige Komponenten, wie gehärtete Ricinolsäure, enthalten können.

Desweiteren ist die Verwendung von Lactonen, wie Butyrolacton oder Caprolacton als Fettsäurekomponente möglich.

In den erfindungsgemäßen Polyglycerinpartialestern sind die Hydroxylgruppen des Polyglycerins zu 20 bis 75 %, bevorzugt 40 bis 70 %, verestert.

Bei ihrer Herstellung werden die Varianten A) und B) bevorzugt gemäß denen in
- A): der ersten Stufe Polyglycerin bis zu einem Veresterungsgrad von 10 bis 70 %, bevorzugt 25 bis 40 %, mit Fettsäure und Dicarbonsäure und/oder Tricarbonsäure verestert wird und dann in einer zweiten Stufe mit Polyricinolsäure zu einem Gesamtveresterungsgrad von 20 bis 75 %, bevorzugt 40 bis 60 %, verestert wird oder in
- B): der ersten Stufe Polyglycerin bis zu einem Veresterungsgrad von 10 bis 70 %, bevorzugt 25 bis 40 %, mit Fettsäure und Polyricinolsäure verestert wird und in einer zweiten Stufe mit Dicarbonsäure und/oder Tricarbonsäure zu einem Gesamtveresterungsgrad von 20 bis 75 %, bevorzugt 40 bis 60 %, verestert wird.

Andere Zugabereihenfolgen der Komponenten, wie z.B. erst Zugabe der Fettsäure, dann der Dicarbonsäure und/oder Tricarbonsäure und anschließend der Polyricinolsäure und Eintopfverfahren sind ebenfalls möglich.

Durch geeignete Auswahl der hydrophilen und lipophilen Molekülanteile kann zum Beispiel ein HLB-Wert von ca. 3 bis 8 eingestellt werden, um für die Stabilisierung von W/O-Emulsionen günstige Eigenschaften zu erhalten.

Die erfindungsgemäßen Polyglycerinpartialester können in an sich bekannter Weise durch Erhitzen der Reaktionskomponenten auf 100 bis 300 °C, vorzugsweise 180 bis 260 °C und destillativer Abtrennung des entstehenden Reaktionswassers hergestellt werden. Zur Beschleunigung können saure oder basische Katalysatoren, wie Sulfonsäuren, Phosphorsäure oder phosphorige Säure, Lewissäuren, wie Zinksalze, Alkali- oder Erdalkalimetalloxide oder -hydroxide, -alkoholate oder -salze mitverwendet werden. Der Zusatz eines Katalysators ist aber nicht unbedingt notwendig. Der fortschreitende Umsatz kann beispielsweise über das abgetrennte Reaktionswasser, durch Messung der Säurezahl oder durch Infrarotspektroskopie verfolgt werden. Im Allgemeinen wird eine Säurezahl im Endprodukt von < 20, bevorzugt < 10 angestrebt. Besonders bevorzugt sind Produkte mit einer Säurezahl < 5.

Die erfindungsgemäßen Polyglycerinester eignen sich insbesondere zur Herstellung von Emulsionen und Dispersionen, bei denen die Ölphase die äußere Phase ist.
Unter bestimmten Voraussetzungen, z.B. durch Zusatz geeigneter hydrophiler Co-Emulgatoren können die erfindungsgemäßen Polyglycerinester auch zur Herstellung von Emulsionen und Dispersionen eingesetzt werden, bei denen die wässrige Phase die äußere Phase bildet.

Bevorzugt ist ihre Verwendung als Emulgatoren und Dispergierhilfsmittel zur Herstellung kosmetischer oder pharmazeutischer Zubereitungen. Dies sind kosmetische Zubereitungen, die durch Verwendung von Öl-in-Wasser- oder Wasser-in-Öl-Emulgatoren eine leicht streichbare Konsistenz erhalten, weil durch diese Emulgatorsysteme sich ein Öl oder ein Fett gut in eine wässrige Phase bzw. eine wässrige Phase gut in ein Öl oder ein Fett einarbeiten lässt, beispielsweise Cremes, wie Pflegecremes, Babycremes oder Sonnenschutzcremes, Salben, Lotionen oder Schminken. In pharmazeutischen Zubereitungen, wie Salben oder Cremes, benötigt man Öl-in-Wasser- oder Wasser-in-Öl-Emulgatoren zur Applikation von Wirkstoffen.

Als kosmetische Öle werden insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen mitverwendet. Ebenso sind im erfindungsgemäßen Sinne die Veresterungsprodukte aliphatischer, difunktioneller Alkohole mit 2 bis 36 C-Atomen mit monofunktionelllen aliphatischen Carbonsäuren mit 1 bis 22 C-Atomen geeignet. Als Ölkomponenten geeignete Monoester sind z. B. die Methylester und Isopropylester von Fettsäuren mit 12 bis 22 C-Atomen wie z. B. Methyllaurat, Methylstearat, Methyloleat, Methylerucat, Isopropylpalmitat, Isopropylmyristat, Isopropylstearat, Isopropyloleat. Andere geeignete Monoester sind z. B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z. B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z. b. im Jojobaöl oder im Spermöl vorliegen.

Mitverwendete Dicarbonsäureester sind z. B. Di-n-butyl-adipat, Di-n-butyl-sebacat, Di-(2-ethylhexyl)-adipat, Di-(2hexyldecyl)-succinat, Diisotridecylacelat. Geeignete Diolester sind z. B. Ethylenglycoldioleat, Ethylenglycol-diisotridecanoat, Propylenglycol-di-(2-ethylhexanoat), Butandiol-di-isostearat und Neopentylglycol-di-caprylat.
Weitere Fettsäureester, die eingesetzt werden können, sind z.B. C₁₂₋₁₅-Alkylbenzoat, Dicaprylylcarbonat, Diethylhexylcarbonat.

Ebenso als Ölkomponente können Fettsäuretriglyceride verwendet werden, wobei unter diesen die natürlich vorkommenden Öle und Fette bevorzugt sind. So kommen beispielsweise natürliche, pflanzliche Öle, z. B. Olivenöl, Sonnenblumenöl, Sojaöl, Erdnußöl, Rapsöl, Mandelöl, Palmöl aber auch die flüssigen Anteil des Kokosöls oder des Palmkernöls sowie tierische Öle wie z. B. Klauenöl, die flüssigen Anteile des Rindertalgs oder auch synthetische Triglyceride von Capryl-Caprinsäure-Gemischen, Triglyceride aus technischer Ölsäure oder aus Palmitinsäure-Ölsäure-Gemischen als Ölkomponenten in Frage.

Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan.

Weiterhin sind auch lineare oder verzweigte Fettalkohole wie Oleylalkohol oder Octyldodecanol, sowie Fettalkoholether wie Dicaprylyl Ether einsetzbar.
Geeignete Siliconöle und -wachse sind z.B. Polydimethylsiloxane, Cyclomethylsiloxane, sowie aryl- oder alkyl- oder alkoxy-substituierte Polymethylsiloxane oder Cyclomethylsiloxane.

Weiterhin können dem Fachmann bekannte, in kosmetischen und pharmazeutischen Anwendungen übliche Hilfsmittel und Zusatzstoffe eingesetzt werden. Dazu gehören beispielsweise Co-Emulgatoren, Konsistenzgeber, Verdicker, Wachse, UV-Lichtschutzfilter, Antioxidantien, Hydrotrope, Deodorant- und Antitransparantwirkstoffe, Insektrepellentien, Selbstbräuner, Konservierungsmittel, Parfümöle, Farbstoffe und biogene Wirkstoffe.

Neben den erfindungsgemäßen Polyglycerinestern können weitere Emulgatoren oder Tenside verwendet werden. Dabei handelt es sich vorzugsweise um nichtionische, anionische, kationische oder amphotere Tenside.
Insbesondere können die erfindungsgemäßen Polyglycerinester in W/O-Emulsionen, die zusätzlich auch dispergierte Feststoffe enthalten können, dazu eingesetzt werden, die Gesamtviskosität der Zubereitung möglichst gering zu halten.

Als nichtionogene Emulgatoren oder Tenside können Verbindungen aus mindestens einer der folgenden Gruppen mitverwendet werden:
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe
- C_{12/18}-Fettsäuremono- und -diester von Anlagerungsprodukten von 1 bis 30 Mol Ethylenoxid an Glycerin
- Glycerinmono- und -diester und Sorbitanmono- und
- diester von gesättigten und ungesättigten Fettsäuren mit 6 bis 22 Kohlenstoffatomen und deren Ethylenoxidanlagerungsprodukte
- Alkylmono- und -oligoglycoside mit 8 bis 22 Kohlenstoffatomen im Alkylrest und deren Ethylenoxidanlagerungsprodukte
- Anlagerungsprodukte von 2 bis 200 Mol Ethylenoxid an Ricinusöl und/oder gehärtetes Ricinusöl
- Partialester auf Basis linearer, verzweigter, ungesättigter bzw. gesättigter C₆₋₂₂-Fettsäuren, Ricinolsäure sowie 12-Hydroxystearinsäure und Glycerin, Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose).
   Dabei ist die Verwendung von Partialestern des Glycerins und des Polylglycerins bevorzugt. Dabei handelt es sich z.B. um Glycerinoleat, Glycerinisostearat, Polyglycerinisostearate, Polyglycerinoleate, Polyglycerinpolyricinoleate, Polyglycerin-Poly-12-hydroxystearate oder Distearoyl Polyglyceryl-3 Dimer Dilinoleat (ISOLAN® PDI, Degussa). Insbesondere in Kombination mit Distearoyl Polyglyceryl-3 Dimer Dilinoleat (ISOLAN® PDI, Degussa) können die erfindungsgemäßen Polyglycerinpartialester in Abhängigkeit vom Mischungsverhältnis dieser beiden Emulgatortypen so eingesetzt werden, dass sowohl dünnflüssige Wasser-in-Öl-Lotionen, als auch reichhaltige Wasser-in-Öl-Cremes erhalten werden können.
- Mono-, Di- und Trialkylphosphate sowie Mono-, Di- und/oder Tri-PEG-alkylphosphate und deren Salze
- Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 14/12 oder 20/20 oder 18/18 oder 17/18 oder 15/15. Besonders geeignet sind hierbei Produkte wie Bis-PEG/PPG-14/14 Dimethicone (mit Cyclopentasiloxan: ABIL® EM 97 (Degussa)) oder PEG/PPG-16/16 PEG/PPG-16/16 Dimethicone (mit Caprylic/Capric Triglyceride: ABIL® Care 85 (Degussa)).
- Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL® EM 90 (Degussa))
- Mischester aus Pentaerythrit, Fettsäuren, Citronensäure und Fettalkohol gemäß DE-11 65 574 und/oder Mischester von Fettsäuren mit 6 bis 22 Kohlenstoffatomen, Methylglucose und Polyolen, vorzugsweise Glycerin oder Polyglycerin.

Auch können anionische Emulgatoren oder Tenside zusätzlich verwendet werden.
Diese enthalten wasserlöslich machende anionische Gruppen wie z.B. eine Carboxylat-, Sulfat-, Sulfonat- oder Phosphat-Gruppe und einen lipophilen Rest. Hautverträgliche anionische Tenside sind dem Fachmann in großer Zahl bekannt und im Handel erhältlich. Dabei handelt es sich insbesondere um Alkylsulfate oder Alkylsphosphate in Form ihrer Alkali, Ammonium- oder Alkanolammoniumsalze, Alklyethersulfate, Alklyethercarboxylate, Acylsarkosinate sowie Sulfosuccinate und Acylglutamate in Form ihrer Alkali oder Ammoniumsalze.

Auch kationische Emulgatoren können als Tenside zugesetzt werden.

Als solche sind insbesondere quartäre Ammoniumverbindungen verwendbar, etwa Alkltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid. Weiterhin können Monoalklyamidoquats wie z.B. Palmitamidopropyltrimethylammoniumchlorid oder entsprechende Dialkylamidoquats eingesetzt werden. Weiterhin können biologisch gut abbaubare quarternäre Esterverbindungen eingesetzt werden, bei denen es sich meist um quaternierte Fettsäureester auf Basis von Mono-, Di- oder Triethanolamin handelt. Weiterhin können Alkylguanidiniumsalze als kationische Emulgatoren beigesetzt sein.

Weiterhin ist es möglich, amphotere Tenside wie z.B. Betaine, Amphoacetate oder Amphopropionate zusammen mit den erfindungsgemäßen Polyglycerinestern einzusetzen.

Weiterhin können die erfindungsgemäßen dünnflüssigen Emulsionen übliche Hilfs- und Zusatzstoffe enthalten wie Verdickungsmittel, UV-Lichtschutzfilter, Antioxidantien, Hydrotrope, Deodorant- und Antitranspirantwirkstoffe, Insektrepellentien, Selbstbräuner, Parfümöle, Farbstoffe und biogene Wirkstoffe.

Zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Vorzugsweise sind diese anorganischen Ölphasenverdicker dabei hydrophob modifiziert. Zur Verdickung/Stabilisierung von kalthergestellten Wasser-in-Öl-Emulsionen können dabei insbesondere Aerosile und/oder Metallsalze von Fettsäuren, wie z.B. Zinkstearat eingesetzt werden.

Unter UV-Lichtschutzfiltern sind organische Substanzen zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen sind z. B. zu nennen:
- 3-Benzylidencampher und dessen Derivate, z. B. 3-(4-Methylbenzyliden)campher
- 4-Aminobenzoesäurederivate, vorzugsweise 4-(Dimethylamino)benzoesäure-2-ethylhexylester, 4-(Dimethylamino)benzoesäure-2-ethylhexylester und 4-(Dimethylamino)benzoesäureamylester
- Ester der Zimtsäure, vorzugsweise 4-Methoxyzimtsäure-2-ethylhexylester, 4-Methoxyzimtsäureisopentylester, 2-Cyan-3-phenyl-zimtsäure-2-ethylhexylester (Octocrylene)
- Ester der Salicylsäure, vorzugsweise Salicylsäure-2-ethylhexylester, Salicylsäure-4-isopropylbenzylester, Salicylsäurehomomenthylester
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- Ester der Benzalmalonsäure, vorzugsweise 4-Methoxybenzalmalonsäuredi-2-ethylhexyester
- Triazinderivate, wie z. B. 2,4,6-Trianilino-(p-carbo-2'-ethyl-1'-hexyloxy)-1,3,5-triazin und Octyltriazon.
- Propan-1,3-dione, wie z. B. 1-(4-tert.Butylphenyl)-3-(4'methoxyphenyl)propan-1,3-dion.

Als wasserlösliche Substanzen können mitverwendet werden:
- 2-Phenylbenzimidazol-5-sulfonsäure und deren Alkali-, Erdalkali-, Ammonium-, Alkylammonium-, Alkanolammonium- und Glucammoniumsalze
- Sulfonsäurederivate von Benzophenon, vorzugsweise 2-Hydroxy-4-methoxybenzophenon-5-sulfonsäure und ihre Salze
- Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'tert. Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoixid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene -bis-{6-(2H-benzotriazole-2-yl)-4-(1,1,3,3-tetramethylbutyl)-phenol} mit einer Partikelgröße von kleiner 200 nm, das z. B. als 50 %ige wässrige Dispersion erhältlich ist.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P. Finkel in SÖFW-Journai 122, 543 (1996) zu entnehmen.

Zur Verbesserung des Fließverhaltens und der Anwendungseigenschaften können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton
- Technische Oligoglyceringemische mit einem Eigenkondensationsgrad von 1,5 bis 10 wie etwa technische Diglyceringemische mit einem Diglyceringehalt von 40 bis 50 Gew.-%
- Methylolverbindungen, wie insbesondere Trimethylolethan, Trimethylolpropan, Trimethylolbutan, Pentaerythrit und Dipentaerythrit
- Niedrigalkylgucoside, insbesondere solche mit 1 bis 4 Kohlenstoffatomen im Alkylrest, wie beispielsweise Methyl- und Butylglucosid
- Zuckeralkohole mit 5 bis 12 Kohlenstoffatomen, wie beispielsweise Sorbit oder Mannit
- Zucker mit 5 bis 12 Kohlenstoffatomen, wie beispielsweise Glucose oder Saccharose
- Aminozucker, wie beispielsweise Glucamin

Als Deodorantwirkstoffe kommen z. B. Geruchsüberdecker wie die gängigen Parfümbestandteile, Geruchsabsorber, beispielsweise die in der Patentoffenlegungsschrift DE-40 09 347 beschriebenen Schichtsilicate, von diesen insbesondere Montmorillonit, Kaolinit, Illit, Beidelit, Nontronit, Saponit, Hectorit, Bentonit, Smectit, ferner beispielsweise Zinksalze der Ricinolsäure in Frage. Keimhemmende Mittel sind ebenfalls geeignet, in die erfindungsgemäßen Öl-in-Wasser-Emulsionen eingearbeitet zu werden. Vorteilhafte Substanzen sind zum Beispiel 2,4,4'-Trichlor-2'-hydroxydiphenylether (Irgasan), 1,6-Di-(4chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO® Cosmo P813, Degussa), sowie die in den Patentoffenlegungsschriften DE-198 55 934, DE-37 40 186, DE-39 38 140, DE-42 04 321, DE-42 29 707, DE-42 29 737, DE-42 38 081, DE-43 09 372, DE-43 24 219 und EP-666 732 beschriebenen wirksamen Agenzien. Weitere übliche Antitranspirantwirkstoffe können ebenfalls vorteilhaft in den erfindungsgemäßen Zubereitungen verwendet werden, insbesondere Adstringentien, beispielsweise basische Aluminiumchloride wie Aluminiumchlorhydrat ("ACH") und Aluminium-Zirkonium-Glycine-Salze ("ZAG").

Als Insekten-Repellentien kommen N,N-Diethyl-m-toluamid, 1,2-Pentandiol oder Insect Repellent 3535 in Frage.

Als Selbstbräuner eignen sich z.B. Dihydroxyaceton und Erythrulose.

Als geeignete Konservierungsmittel können beispielsweise Mischungen einzelner oder mehrerer Alkylparabenester mit Phenoxyethanol eingesetzt. Vorzugsweise handelt es sich bei den Alkylparabenestern um Methlyparaben, Ethylparaben, Propylparaben und/oder Butylparaben. Anstelle von Phenoxyethanol können auch andere Alkohole verwendet werden, wie beispielsweise Benzylalkohol oder Ethanol. Darüber hinaus können auch andere übliche Konservierungsmittel wie etwa Sorbin- oder Benzoesäure, Salicylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate oder die Kombination Chlormethyl-/Methylisothiazolin eingesetzt werden.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z. B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hautpsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Desoxyribonucleinsäure, Coenzym Q10, Retinol, Bisabolol, Allantoin, Phytantriol, Panthenol, AH-Säuren, Aminosäuren, Hyaluronsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte und Vitaminkomplexe zu verstehen.

### Synthesebeispiele:

### Beispiel 1

Die Herstellung des Polyglycerinesters erfolgt in 2 Stufen, die in an sich bekannter Weise ablaufen. Zunächst wird Polyglycerin mit Fettsäure verestert. 94,8 g (0,33 Mol) Isostearinsäure und 17,4 g (0,086 Mol) Sebacinsäure werden mit 67,6 g Polyglycerin in der ersten Stufe bei 240 °C unter Stickstoffdurchleitung verestert. Das Polyglycerin ist durch seine Hydroxylzahl von 1.080 gekennzeichnet. Nach 2 Stunden Reaktionszeit bei dieser Temperatur betrug die Säurezahl < 10. Dann werden bei 240 °C unter Stickstoffdurchleitung 120 g Polyricinolsäure zugegeben. Die Polyricinolsäure ist durch ihre Säurezahl gekennzeichnet. Sie liegt zwischen 100 und 30 bevorzugt zwischen 60 und 40. Anschließend wird der Ansatz solange bei 240 °C gerührt, bis die SZ < 5 beträgt.

### Beispiel 2

Die Herstellung des Polyglycerinesters erfolgt in 2 Stufen, die in an sich bekannter Weise ablaufen. Zunächst wird Polyglycerin mit Fettsäure verestert. 103,8 g (0,36 Mol) Isostearinsäure und 17,4 g (0,086 Mol) Sebacinsäure werden mit 67,6 g Polyglycerin in der ersten Stufe bei 240 °C unter Stickstoffdurchleitung verestert. Das Polyglycerin ist durch seine Hydroxylzahl von 1.080 gekennzeichnet. Nach 2 Stunden Reaktionszeit bei dieser Temperatur betrug die Säurezahl < 10. Dann werden bei 240 °C unter Stickstoffdurchleitung 111 g Polyricinolsäure zugegeben. Die Polyricinolsäure ist durch ihre Säurezahl gekennzeichnet. Sie liegt zwischen 100 und 30 bevorzugt zwischen 60 und 40. Anschließend wird der Ansatz solange bei 240 °C gerührt, bis die SZ < 5 beträgt.

### Beispiel 3

Die Herstellung des Polyglycerinesters erfolgt in 2 Stufen, die in an sich bekannter Weise ablaufen. Zunächst wird Polyglycerin mit Fettsäure verestert. 94,8 g (0,33 Mol) Isostearinsäure und 17,4 g (0,086 Mol) Sebacinsäure werden mit 67,6 g Polyglycerin in der ersten Stufe bei 240 °C unter Stickstoffdurchleitung verestert. Das Polyglycerin ist durch seine Hydroxylzahl von 1.080 gekennzeichnet. Nach 2 Stunden Reaktionszeit bei dieser Temperatur betrug die Säurezahl < 10. Dann werden bei 240 °C unter Stickstoffdurchleitung 96 g Polyricinolsäure zugegeben. Die Polyricinolsäure ist durch ihre Säurezahl gekennzeichnet. Sie liegt zwischen 100 und 30 bevorzugt zwischen 60 und 40. Anschließend wird der Ansatz solange bei 240 °C gerührt, bis die SZ < 5 beträgt.

### Beispiel 4

Die Herstellung des Polyglycerinesters erfolgt in 2 Stufen, die in an sich bekannter Weise ablaufen. Zunächst wird Polyglycerin mit Fettsäure verestert. 120 g (0,42 Mol) Isostearinsäure und 17,4 g (0,086 Mol) Sebacinsäure werden mit 67,6 g Polyglycerin in der ersten Stufe bei 240 °C unter Stickstoffdurchleitung verestert. Das Polyglycerin ist durch seine Hydroxylzahl von 1.080 gekennzeichnet. Nach 2 Stunden Reaktionszeit bei dieser Temperatur betrug die Säurezahl < 10. Dann werden bei 240 °C unter Stickstoffdurchleitung 94,8 g Polyricinolsäure zugegeben. Die Polyricinolsäure ist durch ihre Säurezahl gekennzeichnet. Sie liegt zwischen 100 und 30 bevorzugt zwischen 60 und 40. Anschließend wird der Ansatz solange bei 240 °C gerührt, bis die SZ < 5 beträgt.

### Beispiel 5

Die Herstellung des Polyglycerinesters erfolgt in 2 Stufen, die in an sich bekannter Weise ablaufen. Zunächst wird Polyglycerin mit Fettsäure verestert. 94,8 g (0,33 Mol) Isostearinsäure und 17,4 g (0,086 Mol) Sebacinsäure werden mit 67,6 g Polyglycerin in der ersten Stufe bei 240 °C unter Stickstoffdurchleitung verestert. Das Polyglycerin ist durch seine Hydroxylzahl von 1.140 gekennzeichnet. Nach 2 Stunden Reaktionszeit bei dieser Temperatur betrug die Säurezahl < 10. Dann werden bei 240 °C unter Stickstoffdurchleitung 120 g Polyricinolsäure zugegeben. Die Polyricinolsäure ist durch ihre Säurezahl gekennzeichnet. Sie liegt zwischen 100 und 30 bevorzugt zwischen 60 und 40. Anschließend wird der Ansatz solange bei 240 °C gerührt, bis die SZ < 5 beträgt.

### Beispiel 6

Die Herstellung des Polyglycerinesters erfolgt in 2 Stufen, die in an sich bekannter Weise ablaufen. Zunächst wird Polyglycerin mit Fettsäure verestert. 94,8 g (0,33 Mol) Isostearinsäure und 17,4 g (0,086 Mol) Sebacinsäure werden mit 67,6 g Polyglycerin in der ersten Stufe bei 240 °C unter Stickstoffdurchleitung verestert. Das Polyglycerin ist durch seine Hydroxylzahl von 850 gekennzeichnet. Nach 2 Stunden Reaktionszeit bei dieser Temperatur betrug die Säurezahl < 10. Dann werden bei 240 °C unter Stickstoffdurchleitung 120 g Polyricinolsäure zugegeben. Die Polyricinolsäure ist durch ihre Säurezahl gekennzeichnet. Sie liegt zwischen 100 und 30 bevorzugt zwischen 60 und 40. Anschließend wird der Ansatz solange bei 240°C gerührt, bis die SZ < 5 beträgt.

### Beispiel 7

Die Herstellung des Polyglycerinesters erfolgt in 2 Stufen, die in an sich bekannter Weise ablaufen. Zunächst wird Polyglycerin mit Fettsäure verestert. 103,8 g (0,36 Mol) Isostearinsäure und 17,4 g (0,086 Mol) Sebacinsäure werden mit 67,6 g Polyglycerin in der ersten Stufe bei 240 °C unter Stickstoffdurchleitung verestert. Das Polyglycerin ist durch seine Hydroxylzahl von 1.080 gekennzeichnet. Nach 2 Stunden Reaktionszeit bei dieser Temperatur betrug die Säurezahl < 10. Dann werden bei 240 °C unter Stickstoffdurchleitung 90 g Polyricinolsäure und 22 g Polyhydroxystearinsäure zugegeben. Die Polyricinolsäure ist durch ihre Säurezahl gekennzeichnet. Sie liegt zwischen 100 und 30 bevorzugt zwischen 60 und 40. Anschließend wird der Ansatz solange bei 240 °C gerührt, bis die SZ < 5 beträgt.

### Anwendungsbeispiele:

Die folgenden Beispielemulsionen sollen den Gegenstand der Erfindung näher erläutern, ohne ihn auf diese Beispiele einzuschränken.
Die Konzentrationsangaben in allen Beispielen sind als Gew.-% angegeben.
Wenn nicht anders vermerkt, wurden die Emulsionen so hergestellt, dass die Ölphasen zunächst auf 80 °C erhitzt wurden. Anschließend wurde die Wasserphase in ca. 5 bis 10 Minuten in die Ölphase eingerührt. Nach Beendigung des Einrührens wird kurz homogensiert. Wenn die W/O-Emulsionen unter 30 °C abgekühlt waren, wurde ein zweites Mal homogenisiert.

### Emulsionen 1 bis 6

Die Emulsionen 1 bis 6 sollen insbesondere zeigen, dass es mit Hilfe der erfindungsgemäßen Polyglycerinpartialester möglich war, deutlich niedrigviskosere Lotionen zu erhalten als mit momentan auf dem Markt erhältlichen PEG-freien W/O-Emulgatoren.

Als Emulgatoren des Standes der Technik wurde handelsübliches Polyglyceryl-3-Polyricinoleat und Polyglyceryl-2-Dipolyhydroxystearat eingesetzt.

| | Emulsionen | 1 % | 2 % | 3 % |
|---|---|---|---|---|
| A | Polyglycerinpartialester aus Bsp.1 | 2,00 | | |
| | Polyglyceryl-3-Polyricinoleat | | 2,00 | |
| | Polyglyceryl-2-Dipolyhydroxystearat | | | 2,00 |
| | Hydrogenated Castor Oil | 0,10 | 0,10 | 0,10 |
| | Microcrystalline Wax | 0,10 | 0,10 | 0,10 |
| | Paraffinum Perliquidum | 9,40 | 9,40 | 9,40 |
| | Ethylhexyl Palmitate | 9,40 | 9,40 | 9,40 |
| B | Glycerin | 3,00 | 3,00 | 3,00 |
| | MgSO₄*7H₂O | 1,50 | 1,50 | 1,50 |
| | Bronopol | 0,05 | 0,05 | 0,05 |
| | Water | ad 100 | ad 100 | ad 100 |
| | Stabilität | stabil | stabil | -15°C** |
| | Viskosität* [Pas] | 14 | 28 | 31 |

| | | | | |
|---|---|---|---|---|
| * Brookfield RVT Spindel 5, 10 rpm | | | | |
| ** Wasserseparation bei zwei Gefriertauzyklen 25 °C/-15 °C. | | | | |

| | Emulsionen | 4 % | 5 % | 6 % |
|---|---|---|---|---|
| A | Polyglycerinpartialester aus Bsp.1 | 2,00 | | |
| | Polyglyceryl-3-Polyricinoleat | | 2,00 | |
| | Polyglyceryl-2-Dipolyhydroxystearat | | | 2,00 |
| | Hydrogenated Castor Oil | 0,10 | 0,10 | 0,10 |
| | Microcrystalline Wax | 0,10 | 0,10 | 0,10 |
| | Ethylhexyl Palmitate | 9,40 | 9,40 | 9,40 |
| | Caprylic/Capric Triglyceride | 9,40 | 9,40 | 9,40 |
| B | Glycerin | 3,00 | 3,00 | 3,00 |
| | MgSO₄*7H₂O | 1,50 | 1,50 | 1,50 |
| | Bronopol | 0,05 | 0,05 | 0,05 |
| | Water | ad 100 | ad 100 | ad 100 |
| | Stabilität | stabil | stabil | -15°C** |
| | Viskosität* [Pas] | 21 | 36 | 32 |

| | | | | |
|---|---|---|---|---|
| * Brookfield RVT Spindel 5, 10 rpm | | | | |
| ** Wasserseparation bei zwei Gefriertauzyklen 25 °C/-15 °C. | | | | |

Die Beispielemulsionen zeigen durch die Verwendung unterschiedlicher Ölphasen deutlich, dass ein erfindungsgemäßer Polyglycerinpartialester zu deutlich niedrigeren Emulsionsviskositäten führt als dies mit für den Stand der Technik üblichen PEG-freien W/O-Emulgatoren möglich ist.

Gleichzeitig zeigten die mit Hilfe des erfindungsgemäßen Polyglycerinpartialesters aus Beispiel 1 hergestellten Emulsionen ein ausgezeichnetes leichtes Hautgefühl.

Neben einer excellenten Wärme- und Viskositätsstabilität zeichnet sich der erfindungsgemäße Polyglycerinpartialester aus Beispiel 1 auch durch eine hervorragende Kältestabilisierung der Emulsionen aus.

### Emulsionen 7 bis 10

Die Emulsionen 7 bis 10 sollen insbesondere zeigen, dass es mit Hilfe der erfindungsgemäßen Polyglycerinpartialester möglich ist, mit einer Vielzahl unterschiedlicher Öle stabile dünnflüssige W/O-Emulsionen zu erhalten. Insbesondere Beispielemulsion 10, die größere Mengen Cyclopentasiloxan enthält, zeichnet sich durch ein ausgezeichnetes leichtes Hautgefühl aus.

| | Emulsionen | 7 % | 8 % | 9 % | 10 % |
|---|---|---|---|---|---|
| A | Polyglycerinpartialester aus Bsp. 2 | 2,50 | 2,50 | 2,00 | 2,00 |
| | Hydrogenated Castor Oil | 0,25 | 0,25 | 0,25 | 0,10 |
| | Microcrystalline Wax | 0,25 | 0,25 | 0,25 | 0,10 |
| | Isohexadecane | 5,00 | | | |
| | Cetearyl Ethylhexanoate | 5,00 | 7,00 | | |
| | Ethylhexyl Stearate | 7,00 | | | |
| | Diethylhexyl Carbonate | | 8,00 | 8,50 | 8,90 |
| | Jojoba Oil | | 4,00 | | |
| | Triisostearin | | | 3,00 | |
| | Paraffinum Perliquidum | | | 10,0 | |
| | Cylcopentasiloxane | | | | 8,90 |
| B | Glycerin | 3,00 | 3,00 | 3,00 | 3,00 |
| | MgSO₄*7H₂O | 1,50 | 1,50 | 1,50 | 1,50 |
| | Panthenol | 0,50 | 0,50 | 0,50 | |
| | Bronopol | 0,05 | 0,05 | 0,05 | 0,05 |
| | Ethanol | 3,00 | | | |
| | Water | ad 100 | ad 100 | ad 100 | ad 100 |
| | Stabilität | stable | stable | stable | stable |
| | Viskosität* | 17 | 15 | 12 | 11 |

| | | | | | |
|---|---|---|---|---|---|
| * Brookfield RVT Spindel 5, 10 rpm | | | | | |

## Patentansprüche

1. Polyglycerinpartialester von Polyricinolsäure und mehrfunktionellen Carbonsäuren, erhältlich durch Veresterung
a) eines Polyglyceringemisches mit
b) mindestens einer Polyricinolsäure der allgemeinen Formel 1 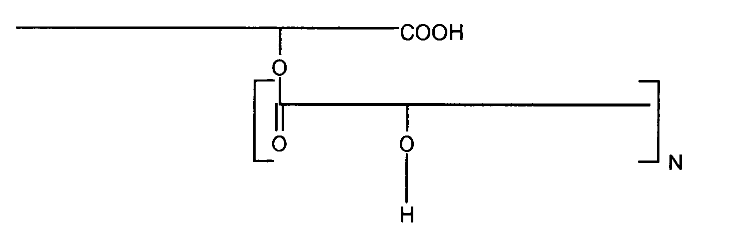 worin 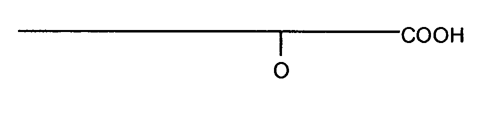 der Rest der Ricinolsäure CH₃-(CH₂)₅-CH(OH)-CH₂-CH=CH-(CH₂)₇-COOH und gegebenenfalls der Hydroxystearinsäure CH₃-(CH₂)₅-CH(OH)-(CH₂)₁₀-COOH ist,
N 1 bis 10 bedeutet und gegebenenfalls
b1) Polyhydroxystearinsäure und
c) mindestens einer Di- und/oder Tricarbonsäure nach an sich bekannten Verfahren,
**dadurch gekennzeichnet, dass**
in der Veresterung zusätzlich
d) mindestens eine Fettsäure oder ein Lacton als Fettsäurekomponente
eingesetzt wird.

2. Polyglycerinpartialester gemäß Anspruch 1, dadurch gekennzeichnen, dass die Polyglyceringemische gemäß a) einen mittleren Kondensationsgrad von 1 bis 11, vorzugsweise 2 bis 6 aufweisen.

3. Polyglycerinpartialester gemäß einem der Ansprüche 1 oder 2, **dadurch gekennzeichnet, dass** die Polyricinolsäure gemäß b) einen mittleren Kondensationsgrad von 1 bis 10, vorzugsweise 2 bis 8 aufweist.

4. Polyglycerinpartialester gemäß mindestens einem der Ansprüche 1 bis 3, **dadurch gekennzeichnet, dass** die Di- und/oder Tricarbonsäuren gemäß c) aliphatische, lineare oder verzweigte Di- und/oder Tricarbonsäuren sind und 2 bis 36 C-Atome, vorzugsweise 3 bis 12 C-Atome aufweisen.

5. Polyglycerinpartialester gemäß mindestens einem der Ansprüche 1 bis 4, **dadurch gekennzeichnet, dass** die Fettsäuren gemäß d) gesättigte oder ungesättigte, lineare oder verzweigte einbasische Säuren mit 6 bis 22 C-Atomen sind.

6. Polyglycerinpartialester gemäß mindestens einem der Ansprüche 1 bis 5, **dadurch gekennzeichnet, dass** zwischen 20 und 75 % der OH-Gruppen der Polyglycerinmischung verestert sind.

7. Polyglycerinpartialester gemäß mindestens einem der Ansprüche 1 bis 6, hergestellt durch Veresterung von
a) 1,0 Mol OH-Gruppen des Polyglyceringemisches mit
b) 0,01 bis 0,5 Mol COOH-Gruppen der Polyricinolsäure und
c) 0,01 bis 0,5 Mol COOH-Gruppen mindestens einer Di- und/oder Tricarbonsäure und
d) 0,01 bis 0,9 Mol COOH-Gruppen der Fettsäuren mit 6 bis 22 C-Atomen,
mit der Maßgabe, das die Summe der COOH-Gruppen etwa 20 bis 75 % der OH-Gruppen des Polyglyceringemisches entspricht.

8. Polyglycerinpartialester gemäß mindestens einem der Ansprüche 1 bis 6, hergestellt durch Veresterung von
a) 1,0 Mol OH-Gruppen des Polyglyceringemisches mit
b) 0,01 bis 0,5 Mol COOH-Gruppen der Polyricinolsäure und
c) 0,01 bis 0,5 Mol COOH-Gruppen mindestens einer Di- und/oder Tricarbonsäure,
mit der Maßgabe, das die Summe der COOH-Gruppen etwa 20 bis 75 % der OH-Gruppen des Polyglyceringemisches entspricht.

9. Verfahren zur Herstellung der Polyglycerinpartialester gemäß Anspruch 1, **dadurch gekennzeichnet, dass** in
der ersten Stufe Polyglycerin bis zu einem Veresterungsgrad von 10 bis 70 % mit Fettsäure oder Lacton als Fettsäurekomponente und Di- und/oder Tricarbonsäuren verestert und in
der zweiter Stufe mit Polyricinolsäure zu einem Gesamtveresterungsgrad von 20 bis 75 % verestert wird oder dass in
der ersten Stufe Polyglycerin bis zu einem Veresterungsgrad von 10 bis 70 % mit Fettsäure oder Lacton als Fettsäurekomponente und Polyricinolsäure verestert und in
der zweiter Stufe mit Di- und/oder Tricarbonsäuren zu einem Gesamtveresterungsgrad von 20 bis 75 % verestert wird.

10. Kosmetische, dermatologische oder pharmazeutische Zubereitungen, die mindestens einen der Polyglycerinpartialester gemäß mindestens einem der Ansprüche 1 bis 8 sowie gegebenenfalls übliche Hilfs- und Zusatzstoffe enthalten.

11. Pflege- und Reinigungsmittel für haushalts und industrielle Anwendungen die mindestens einen der Polyglycerinpartialester gemäß mindestens einem der Ansprüche 1 bis 8 sowie gegebenenfalls übliche Hilfs- und Zusatzstoffe enthalten.

## Claims

1. Polyglycerol partial esters of polyricinoleic acid and polyfunctional carboxylic acids, obtainable by esterification
a) of a polyglycerol mixture with
b) at least one polyricinoleic acid of the general formula 1 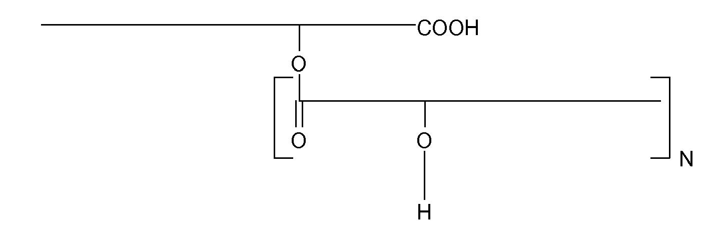 in which 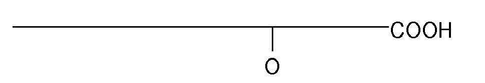 is the radical of ricinoleic acid CH₃-(CH₂)₅-CH(OH)-CH₂-CH=CH-(CH₂)₇-COOH and optionally of hydroxystearic acid CH₃-(CH₂)₅-CH(OH)-(CH₂)₁₀-COOH,
N is 1 to 10 and optionally
b1) polyhydroxystearic acid and
c) at least one di- and/or tricarboxylic acid by methods known per se,
**characterized in that**
in the esterification additionally
d) at least one fatty acid or a lactone as fatty acid component
is used.

2. Polyglycerol partial esters according to Claim 1, **characterized in that** the polyglycerol mixtures according to a) have an average degree of condensation of from 1 to 11, preferably 2 to 6.

3. Polyglycerol partial esters according to either of Claims 1 and 2, **characterized in that** the polyricinoleic acid according to b) has an average degree of condensation of from 1 to 10, preferably 2 to 8.

4. Polyglycerol partial esters according to at least one of Claims 1 to 3, **characterized in that** the di- and/or tricarboxylic acids according to c) are aliphatic, linear or branched di- and/or tricarboxylic acids and have 2 to 36 carbon atoms, preferably 3 to 12 carbon atoms.

5. Polyglycerol partial esters according to at least one of Claims 1 to 4, **characterized in that** the fatty acids according to d) are saturated or unsaturated, linear or branched monobasic acids having 6 to 22 carbon atoms.

6. Polyglycerol partial esters according to at least one of Claims 1 to 5, **characterized in that** between 20 and 75% of the OH groups of the polyglycerol mixture are esterified.

7. Polyglycerol partial esters according to at least one of Claims 1 to 6, prepared by esterification of
a) 1.0 mol of OH groups of the polyglycerol mixture with
b) 0.01 to 0.5 mol of COOH groups of the polyricinoleic acid and
c) 0.01 to 0.5 mol of COOH groups of at least one di- and/or tricarboxylic acid and
d) 0.01 to 0.9 mol of COOH groups of the fatty
acids having 6 to 22 carbon atoms,
with the proviso that the sum of the COOH groups corresponds to about 20 to 75% of the OH groups of the polyglycerol mixture.

8. Polyglycerol partial esters according to at least one of Claims 1 to 6, prepared by esterification of
a) 1.0 mol of OH groups of the polyglycerol mixture with
b) 0.01 to 0.5 mol of COOH groups of the polyricinoleic acid and
c) 0.01 to 0.5 mol of COOH groups of at least one di- and/or tricarboxylic acid,
with the proviso that the sum of the COOH groups corresponds to about 20 to 75% of the OH groups of the polyglycerol mixture.

9. Method of producing the polyglycerol partial esters according to Claim 1, **characterized in that**, in
the first stage, polyglycerol is esterified to a degree of esterification from 10 to 70% with fatty acid or lactone as fatty acid component and di- and/or tricarboxylic acids and in
the second stage is esterified with polyricinoleic acid to a total degree of esterification of from 20 to 75%, or **in that** in
the first stage polyglycerol is esterified to a degree of esterification of from 10 to 70% with fatty acid or lactone as fatty acid component and polyricinoleic acid and in
the second stage is esterified with di- and/or tricarboxylic acids to a total degree of esterification of from 20 to 75%.

10. Cosmetic, dermatological or pharmaceutical preparation which comprises at least one of the polyglycerol partial esters as claimed in at least one of Claims 1 to 8, and optionally customary auxiliaries and additives.

11. Care and cleaning composition for household and industrial applications which comprises at least one of the polyglycerol partial esters as claimed in at least one of Claims 1 to 8, and optionally customary auxiliaries and additives.

## Revendications

1. Esters partiels de polyglycérol de l'acide polyricinoléique et d'acides carboxyliques polyfonctionnels, pouvant être obtenus par estérification
a) d'un mélange de polyglycérols avec
b) au moins un acide polyricinoléique de formule générale 1 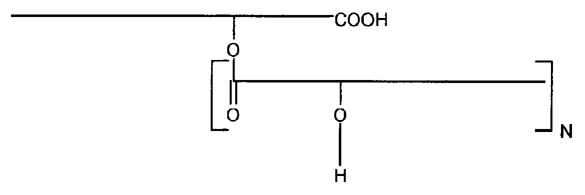 où 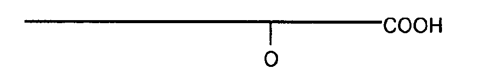 est le radical de l'acide ricinoléique CH₃-(CH₂)₅-CH(OH)-CH₂-CH=CH-(CH₂)₇-COOH et le cas échéant de l'acide hydroxystéarique CH₃-(CH₂)₅-CH(OH)-(CH₂)₁₀-COOH,
N vaut 1 à 10 et le cas échéant
b1) de l'acide polyhydroxystéarique et
c) au moins un acide dicarboxylique et/ou tricarboxylique selon des procédés connus en soi, **caractérisés**
**en ce qu'**on utilise, dans l'estérification, en plus
d) au moins un acide gras ou une lactone comme composant d'acide gras.

2. Esters partiels de polyglycérol selon la revendication 1, **caractérisés en ce que** les mélanges de polyglycérols selon a) présentent un degré de condensation moyen de 1 à 11, de préférence de 2 à 6.

3. Esters partiels de polyglycérol selon l'une quelconque des revendications 1 ou 2, **caractérisés en ce que** l'acide polyricinoléique selon b) présente un degré de condensation moyen de 1 à 10, de préférence de 2 à 8.

4. Esters partiels de polyglycérol selon au moins l'une quelconque des revendications 1 à 3, **caractérisés en ce que** les acides dicarboxyliques et/ou tricarboxyliques selon c) sont des acides dicarboxyliques et/ou tricarboxyliques aliphatiques, linéaires ou ramifiés et présentent 2 à 36 atomes de carbone, de préférence 3 à 12 atomes de carbone.

5. Esters partiels de polyglycérol selon au moins l'une quelconque des revendications 1 à 4, **caractérisés en ce que** les acides gras selon d) sont des acides saturés ou insaturés, linéaires ou ramifiés, monobasiques comprenant 6 à 22 atomes de carbone.

6. Esters partiels de polyglycérol selon au moins l'une quelconque des revendications 1 à 5, **caractérisés en ce qu'**entre 20 et 75% des groupes OH du mélange de polyglycérols sont estérifiés.

7. Esters partiels de polyglycérol selon au moins l'une quelconque des revendications 1 à 6, préparés par estérification de
a) 1,0 mole de groupes OH du mélange de polyglycérols avec
b) 0,01 à 0,5 mole de groupes COOH de l'acide polyricinoléique et
c) 0,01 à 0,5 mole de groupes COOH d'au moins un acide dicarboxylique et/ou tricarboxylique et
d) 0,01 à 0,9 mole de groupes COOH des acides gras comprenant 6 à 22 atomes de carbone, à condition que la somme des groupes COOH corresponde à environ 20 jusqu'à 75% des groupes OH du mélange de polyglycérols.

8. Esters partiels de polyglycérol selon au moins l'une quelconque des revendications 1 à 6, préparés par estérification de
a) 1,0 mole de groupes OH du mélange de polyglycérols avec
b) 0,01 à 0,5 mole de groupes COOH de l'acide polyricinoléique et
c) 0,01 à 0,5 mole de groupes COOH d'au moins un acide dicarboxylique et/ou tricarboxylique, à condition que la somme des groupes COOH corresponde à environ 20 jusqu'à 75% des groupes OH du mélange de polyglycérols.

9. Procédé pour la préparation des esters partiels de polyglycérol selon la revendication 1, **caractérisé en ce qu'**on estérifie dans la première étape le polyglycérol jusqu'à un degré d'estérification de 10 à 70% avec un acide gras ou une lactone comme composant acide gras et des acides dicarboxyliques et/ou tricarboxyliques et dans la deuxième étape avec de l'acide polyricinoléique jusqu'à un degré total d'estérification de 20 à 75% ou **en ce qu'**on estérifie dans la première étape le polyglycérol jusqu'à un degré d'estérification de 10 à 70% avec un acide gras ou une lactone comme composant acide gras et de l'acide polyricinoléique et dans la deuxième étape avec des acides dicarboxyliques et/ou tricarboxyliques jusqu'à un degré total d'estérification de 20 à 75%.

10. Préparations cosmétiques, dermatologiques ou pharmaceutiques, qui contiennent au moins un des esters partiels de polyglycérol selon au moins l'une quelconque des revendications 1 à 8 ainsi que le cas échéant des adjuvants et additifs usuels.

11. Agents d'entretien et de nettoyage pour des utilisations domestiques et industrielles qui contiennent au moins un des esters partiels de polyglycérol selon au moins l'une quelconque des revendications 1 à 8 ainsi que le cas échéant des adjuvants et additifs usuels.
